# EUROPEAN PATENT SPECIFICATION

(11) **EP 0 860 439 B1**
(45) Date of publication and mention of the grant of the patent: **13.06.2001**
(21) Application number: 98200484.8
(22) Date of filing: 16.02.1998
(51) Int. Cl.: C07D 303/48, C07D 281/10, C07C 219/14

(54) **Process for preparing an optically active phenylglycidyl acid derivative**
Verfahren zur Herstellung eines optisch aktiven Phenylglycidylsäurederivates
Procédé pour la préparation d'un dérivé optiquement actif d'acide phénylglycidique

(30) Priority: 21.02.1997 NL 1005338
(43) Date of publication of application: 26.08.1998
(73) Proprietor: DSM N.V., 6411 TE Heerlen (NL)
(72) Inventor: Kaptein, Bernardus, 6133 BE Sittard (NL); Verzijl, Gerardus Karel Maria, 5855 AR Bergen (NL)

(56) References cited:
- EP-A- 0 342 904
- A. SCHWARTZ ET AL.: "Enantioselective synthesis of calcium channel blockers of the diltiazem group" JOURNAL OF ORGANIC CHEMISTRY, vol. 57, no. 3, 1992, WASHINGTON DC, US, pages 851-6, XP002042991
- A.K. GHOSH ET AL.: "Highly stereoselective reduction of alpha-keto esters: utility of cis-1-arylsulfonamido- 2-indanols as chiral auxiliaries" TETRAHEDRON LETTERS, vol. 36, no. 38, 18 September 1995, OXFORD, GB, pages 6811-4, XP004027392

## Description

The invention relates to a process for preparing an optically active trans-phenylglycidic acid derivative having formula (1), 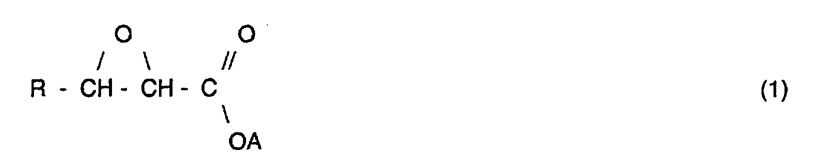 in which R represents a phenyl group, whether or not substituted, and A is derived from an optically active compound, in which an aldehyde having formula (2), 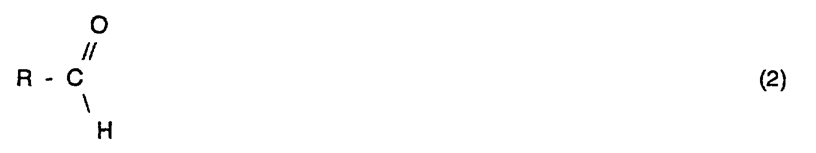 in which R is as defined above, is, in the presence of a base, brought into contact with an optically active acetyl compound having formula (3), 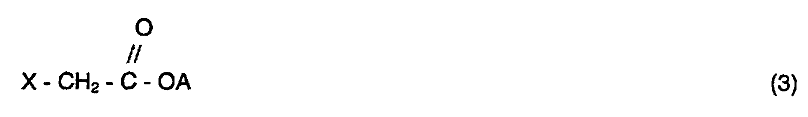 in which X represents a leaving group, characterized in that use is made of an optically active compound having formula (3) in which A is derived from an amino alcohol.

A similar process wherein an optically active trans-phenylglycidic derivate with formula (1) is prepared in which A represents (-)-8-phenylmenthyl chloroacetate is known from EP-A-342904.

An optically active trans-phenylglycidic derivate with formula (1) is also prepared in A. Schwartz et al., Journal of Organic Chemistry, Vol. 57, no. 3, pages 851-856 (1992), in which A is derived from a 2-phenylcyclohexanol.

The process according to the invention provides an alternative process wherein a high yield of the desired enantiomer and/or a high diastereomeric ratio can be obtained. Diastereoisomeric ratio means the (molar or weight) ratio between two diastereomeric isomers.

As the aldehyde use is made of an aldehyde having formula (2), in which R represents a phenyl group which may in one or more places be substituted with, for example, an alkyl group or an alkoxy group preferably having 1-20 C atoms, in particular 1-5 C atoms.

As the acetyl compound use is made of a compound having formula (3), in which X represents a leaving group and A a chiral group derived from an optically active amino alcohol (AOH). Groups that can be used as the leaving group are commonly known from the literature. Very suitable leaving groups are for example halogenides, in particular Cl⁻ or Br⁻, sulphonates, for example p-toluene or methane sulphonate. Amino alcohols from which A may be derived are for example (salts of) a β-amino alcohol which preferably has a more or less rigid structure, for example because of the amino alcohol containing a ring structure. Particularly suitable examples of amino alcohols are substituted amino indanols having formula (4), 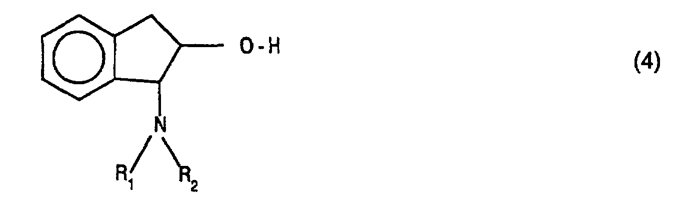 in which R₁ and R₂ represent a (hetero)alkyl, an alkenyl, a (hetero)aryl or an arylsulphonyl group, whether or not substituted, having 1-10 C atoms, or R₁ and R₂ constitute an aromatic or aliphatic ring together with the N atom to which they are bound. The best results were obtained when use was made of a haloacetyl compound having formula (3), in which X represents Cl and A is derived from an enantiomerically pure (i.e. having an enantiomeric excess (e.e.) > 95%, in particular > 99%) cis-amino indanol having formula (4), in which R₁ and R₂ are each independently methyl, ethyl, isopropyl, n-propyl, n-butyl, allyl, benzyl or tosyl.

Preferably a non-nucleophilic base is used as the base, for example a hydride, in particular potassium hydride or sodium hydride; an alkyl lithium, more in particular n-butyl lithium, or an alkoxide, preferably potassium t-butoxide. Preferably use is made of potassium t-butoxide.

As the solvent, use is made of for example chlorinated hydrocarbons, aromatic hydrocarbons or ethers that are inert in the reaction system, for example dichloromethane, toluene, xylene or tetrahydrofuran (THF). Preferably use is made of toluene or dichloromethane.

The temperature at which the reaction is carried out is not particularly critical and preferably lies between -30 and 50°C, in particular between 10 and 40°C.

The molar ratio of the aldehyde and the acetyl compound is not critical either, and in practice preferably lies between 1:2 and 2:1, in particular between 1:1.1 and 1.1:1, with a virtually equimolar ratio seeming optimum.

Preferably use is made of a slight excess of base relative to the acetyl compound, for example a base : acetyl compound molar ratio of between 1:1 and 1.5:1, preferably between 1:1 and 1.1:1. It will be clear that when A is derived from a salt of an optically active amino alcohol, for example the HCl salt, an extra equivalent of the base will need to be used.

Optically active phenylglycidyl acid derivatives obtained with the process according to the invention can be used with particular advantage in the preparation of pharmaceuticals, in particular benzothiazepines, for example diltiazem and clenthiazem. The invention also relates to optically active phenylglycidyl acid derivatives having formula (1) and to the use thereof in the preparation of such pharmaceuticals.

In particular, the invention also relates to the new optically active compounds having formula (1) in which R represents a phenyl group, whether or not substituted, as defined above and A is derived from an optically active cis-amino indanol (AOH) according to formula (4), in which R₁ and R₂ represent a (hetero)alkyl, an alkenyl, a (hetero)aryl or an arylsulphonyl group, whether or not substituted, having 1-10 C atoms, or R₁ and R₂ constitute an aromatic or aliphatic ring together with the N atom to which they are bound, R₁ and R₂ each independently preferably representing methyl, ethyl, isopropyl, n-propyl, n-butyl, allyl, benzyl or tosyl; and to the optically active acetyl compounds having formula (3), in which X represents a leaving group and A is as defined above.

The optically active compounds having formula (1) can be used as such directly in the preparation of pharmaceuticals or they can first be converted into a corresponding phenylglycidyl ester, for example the methyl, ethyl or t-butyl ester, for instance by reaction with a base and an alcohol e.g. an alkalimetal alkoxide with the desired ester corresponding alcohol as solvent. These phenylglycidyl esters can in turn be converted into pharmaceuticals in a known manner, for example through coupling with an optionally substituted 2-amino thiophenol and cyclisation to a benzothiazepine. Such benzothiazepines are intermediates in the preparation of known pharmaceuticals, for example diltiazem and clenthiazem. For the preparation of diltiazem use is made of for example the (2R,3S) compound having formula (1), in which R represents p-methoxyphenyl and A is derived from (1S, 2R)-amino indanol, or a corresponding p-methoxyphenylglycidyl ester obtained therefrom is first brought into contact with for example a 2-amino thiophenol, after which the reaction product obtained is subjected to a cyclisation reaction, optionally followed by an alkylation and acylation reaction.

The invention will now be further elucidated with reference to the examples, without being limited thereto.

### Example I

### (1S,2R)-1-(Diethylamino)-2-indanol

A suspension of 15.09 grams (101 mmol) of (1S,2R)-1-amino-2-indanol, 33.72 grams (244 mmol) of potassium carbonate and 38.8 grams (249 mmol) of ethyl iodide in 100 ml of acetonitrile was refluxed for 3 hours. After cooling, the solid matter was removed through filtration and the filtrate was evaporated. The residue was dissolved in 4N hydrochloric acid and was washed three times using dichloromethane. The water layer was made basic with the aid of a 50% sodium hydroxide solution and was extracted with the aid of dichloromethane (3*25 ml). After drying (Na₂SO₄) and evaporation, the product was isolated as an oil. This oil was dissolved in diethyl ether and cooled, which caused the product to crystallise.
Yield: 16.7 grams (81%) of a white solid substance. Melting point: 60-61°C. [α]²⁰_{D} +1.1 (c=1, methanol). ¹H NMR (200 MHz, CDCl₃) : 1.02 (t, 6H), 2.24-2.57 (2*m, 4H), 2.65 (dd, 1H), 3.20 (dd, 1H), 4.22 (d, 1H), 4.29 (q, 1H), 4.7 (br s, 1H) and 7.10-7.27 (m, 4H). ¹³C NMR (50.31 MHz, CDCl₃) : 13.63 (q), 41.42 (t) , 45.60 (t), 66.97 (d), 68.95 (d), 125.44 (d), 126.17 (d), 126.45 (d), 128.28 (d), 139.65(s) and 141.98 (s).

### Example II

### (1S,2R)-N-Methyl,N-i-propyl-1-amino-2-indanol

A solution of 4.0 grams of (1S,2R)-1-(i-propylamino)-2-indanol (20.9 mmol), 30 ml of formic acid and 15 g of Formalin was refluxed for 24 hours. After cooling, the reaction mixture was evaporated and dissolved in 50 ml of an aqueous 4N sodium hydroxide solution. The basic solution was extracted with the aid of dichloromethane (2*50 ml). After drying (Na₂SO₄) and evaporation, the oil was chromatographed using silica gel 60 (eluant: ethylacetate/petroleum ether (40-70) 3:1).
Yield: 3.4 grams (79%) of a colourless oil. [α]²⁰_{D}+8.6 (c=1, methanol). ¹H NMR (200 MHz, CDCl₃) : 0.95 (d, 6H), 1.67 (s, 3H), 2.48 (dd, J=8.3 and 3.5 Hz, 1H), 2.83 (septet, 1H), 3.02 (dd, J=8.3 and 3.9 Hz, 1H), 3.98-4.12 (m, 2H), 4.9 (br s, 1H) and 6.94-7.08 (m, 4H).¹³C NMR (50.31 MHz, CDCl₃): 21.40 (q), 21.76 (q), 35.67 (q), 42.86 (t), 56.51 (d), 66.70 (d), 70.70 (d), 126.99 (d), 127.81 (d), 128.40 (d), 129.66 (d), 140.63 (s) and 143.67 (s).

### Example III

### (1S,2R)-1-(Diethylamino)-2-indanyl chloroacetate

1.70 grams (17 mmol) of chloroacetyl chloride was added, drop by drop, in 5 minutes, to a solution of 2.65 grams (13 mmol) of (1S,2R)-1-(diethylamino)-2-indanol in 40 ml of dichloromethane at room temperature. The temperature increased to 30°C. The reaction was stirred for 15 hours at room temperature. After evaporation of the solution, 4.15 grams (91%) of HCl salt was obtained as a yellow foam. ¹H NMR (200 MHz, CDCl₃): 1.27 (t, 3H), 1.49 (t, 3H), 2.80 (septet, 1H), 2.96-3.39 (m, 5H), 4.16 (d, J=7.8 Hz, 1H), 4.41 (d, J=7.8 Hz, 1H), 5.04 (br d, 1H), 5.78 (q, 1H), 7.15-7.37 (m, 3H), 7.55 (d, 1H) and 11.5 (br s, 1H).
The HCl salt can be quantitatively liberated to yield a colourless oil through extraction using a dichloromethane/5% K₂CO₃ solution in water. ¹H NMR (200 MHz, CDCl₃): 0.97 (t, 6H), 2.40-2.71 (m, 4H), 2.93 (dd, 1H), 3.12 (dd, 1H), 3.96 (s, 2H), 4.50 (d,1H), 5.56 (ddd, 1H) and 7.15-7.30 (m, 4H). ¹³C NMR (50.31 MHz, CDCl₃) : 14.74 (q), 37.51 (t), 41.25 (t), 45.25 (t), 66.64 (d), 77.89 (d), 124.92 (d), 125.26 (d), 126.89 (d), 127.76 (d), 139.07 (s), 141.20 (s) and 167.03 (s).

### Example IV

### (1S,2R)-N-Methyl,N-i-propyl-1-amino-2-indanyl chloroacetate

2.00 grams (17.5 mmol) of chloroacetyl chloride was added drop by drop, in 5 minutes, to a solution of 3.00 grams (14.6 mmol) of (1S,2R)-N-methyl,N-i-propyl-1-amino-2-indanol in 40 ml of dichloromethane at room temperature. The temperature increased to 35°C. The reaction was stirred for 15 hours at room temperature. This was followed by the addition of 50 ml of a 5% K₂CO₃ solution in water and extraction. The basic water layer was once again extracted using 30 ml of dichloromethane. After the collected organic layers had been washed with water, dried using Na₂SO₄ and evaporated, 3.96 grams (85%) of product was isolated as a yellow oil. ¹H NMR (200 MHz, CDCl₃) : 1.05 (2*d, 6H), 2.09 (s, 3H), 2.96 (dd, J= 8.6 and 3.5 Hz +septet, 2H), 3.14 (dd, J= 8.6 and 3.5 Hz, 1H), 3.99 (s, 2H), 4.47 (d, J=3,1 Hz, 1H), 5.47 (dt, J=3,1 and 3.5 Hz, 1H) and 7.10-7.25 (m, 4H).
¹³C NMR (50.31 MHz, CDCl₃): 20.59 (q), 33.00 (q), 37.06 (t), 41.10 (t), 53.61 (d), 66.08 (d), 78.47 (d), 124.84 (d), 125.81 (d), 126.62 (d), 127.61 (d), 138.96 (s), 140.46 (s) and 166.85 (s).

### Example V

### (2R,3S)-3-(4-Methoxyphenyl)oxirane-2-carboxylic (1S,2R)-1-(diethylamino)-2-indanyl ester

In 5-10 min., 0.95 grams (8.5 mmol) of potassium tert.-butoxide was added, in small portions, to a solution of 2.05 grams (7.3 mmol) of (1S,2R)-1-(diethylamino)-2-indanyl chloroacetate and 1.0 gram (7.3 mmol) of p-anisic aldehyde in 40 ml of toluene at 20 °C. The temperature increased to 25 °C. After 30 minutes' stirring the reaction was quenched using a diluted NaHCO₃ solution in water. The organic layer was separated and washed with water, dried using Na₂SO₄ and evaporated. Yield: 2.75 grams (90%) of a yellow oil. This oil is a mixture of (2R,3S)-3-(4-methoxyphenyl)oxirane-2-carboxylic (1S,2R)-1-(diethylamino)-2-indanyl ester (diastereomeric ratio 89:11) and (2R,3S)-3-(4-methoxyphenyl)oxirane-2-carboxylic tert.-butyl ester (enantiomeric excess 80%). ¹H NMR (200 MHz, CDCl₃): 1.02 (2*t, 6H), 2.48-2.78 (m,4H), 2.95-3.27 (m, 2H), 3.44 (d, J = 2.0 Hz) and 3.48 (d, J = 2.0 Hz, together 1H for major and minor diastereomers, resp.), 3.80 (s, 3H), 4.02 (d, J = 2.0 Hz) and 4.07 (d, J = 2.0 Hz, together 1H for minor and major diastereomers, resp.), 4.60 (d, 1H), 5.62-5.75 (m, 1H), 6.85 (d, 2H) and 7.15-7.38 (d+m, 6H). ¹³C NMR (50.31 MHz, CDCl₃): 14.71 (q), 37.58 (t), 45.35 (t), 55.30 (q), 56.85 (d), 57.97 (d), 66.51 (d), 77.02 (d), 113.49 (d), 114.10 (d), 124.93 (d), 125.32 (d), 126.81 (d), 127.12 (d), 127.27 (d), 127.74 (s), 139.16 (s), 160.18 (s) and 168.16 (s).
(2R,3S)-3-(4-Methoxyphenyl)oxirane-2-carboxylic tert.-butyl ester: ¹H NMR (200 MHz, CDCl₃): 1.43 (s, 9H), 3.33 (d, 1H), 3.73 (s, 3H), 3.89 (d, 3H), 6.81 (d, 2H) and 7.14 (d, 2H).

### Example VI

### (2R,3S)-3-(4-Methoxyphenyl)oxirane-2-carboxylic (1S,2R)-1-(diethylamino)-2-indanyl ester

In 5-10 min., 3.50 grams (31 mmol) of potassium tert.-butoxide was added, in small portions, to a solution of 4.9 grams (12.5 mmol) of (1S,2R)-1-(diethylamino)-2-indanyl chloroacetate HCl salt and 1.70 grams (12.5 mmol) of p-anisic aldehyde in 50 ml of dichloromethane at 20 °C. The temperature increased to 30 °C. After 60 minutes' stirring the reaction was quenched using a 0.5M KH₂PO₄ solution in water. The organic layer was separated and washed with water, dried using Na₂SO₄ and evaporated. Yield: 5.10 grams (99%) of a brown oil (a mixture of (2R,3S)-3-(4-methoxyphenyl)oxirane-2-carboxylic (1S,2R)-1-(diethylamino)-2-indanyl ester (diastereomeric ratio 89:11) and (2R,3S)-3-(4-methoxyphenyl)oxirane-2-carboxylic tert.-butyl ester (enantiomeric excess 75%)).

### Example VII

### (2R,3S)-3-(4-Methoxyphenyl)oxirane-2-carboxylic (1S,2R)-N-methyl,N-i-propyl-1-amino-2-indanyl ester

In 5 min., 1.50 grams (13.4 mmol) of potassium tert.-butoxide was added, in small portions, to a solution of 3.82 grams (12.0 mmol) of (1S,2R)-N-methyl,N-i-propyl-1-amino-2-indanyl chloroacetate and 1.63 grams (12.0 mmol) of p-anisic aldehyde in 50 ml of toluene at 20 °C. The temperature increased to 33 °C. After 30 minutes' stirring the reaction was quenched with the aid of a 1M NaHCO₃ solution in water. The organic layer was separated and washed with water, dried with the aid of Na₂SO₄ and evaporated. Yield: 4.90 grams (98%) of a yellow oil (a 77:23 mixture of (2R,3S)-3-(4-methoxyphenyl)oxirane-2-carboxylic (1S,2R)-N-methyl,N-i-propyl-1-amino-2-indanyl ester (diastereomeric ratio 79:21) and (2R,3S)-3-(4-methoxyphenyl)oxirane-2-carboxylic tert.-butyl ester). ¹H NMR (200 MHz, CDCl₃): 1.12 (2*d, 6H), 2.21 (s, 3H), 3.05-3.38 (m, 3H), 3.52 (d, J = 2.0 Hz, 0.95H), 3.56 (d, J = 2.0 Hz, 0.05H), 3.79 (s, 3H), 4.05 (d, J =2.0 Hz, 1H), 4.60 (d, 1H), 5.63 (m, 1H), 6.91 (d, 2H) and 7.18-7.37 (d+m, 6H). ¹³C NMR (50.31 MHz, CDCl₃) : 20.53 (q), 20.80 (q), 33.35 (q), 37.17 (t), 53.34 (d), 55.23 (q), 56.76 (d), 57.83 (d), 66.22 (d), 78.16 (d), 114.02 (d), 124.89 (d), 125.81 (d), 126.62 (d), 127.02 (d), 127.56 (d), 127.91 (s), 139.06 (s), 141.0 (s), 160.08 (s) and 167.90 (s).

### Example VIII

### (2S,3S)-2,3-Dihydro-3-hydroxy-2-(4-methoxyphenyl)-1,5-benzothiazepin-4(5H)-on

2.0 grams (4.8 mmol) of the mixture of (2R,3S)-3-(4-methoxyphenyl)oxirane-2-carboxylic (1S,2R)-1-(diethylamino)-2-indanyl ester (diastereomeric ratio 89:11) and (2R,3S)-3-(4-methoxyphenyl)oxirane-2-carboxylic tert.-butyl ester (enantiomeric excess 80%) of Example V was dissolved in 20 ml of xylene + 1 ml of methanol. This solution was heated to 120 °C and 600 mg (5.0 mmol) of 2-aminothiophenol was added drop by drop in 5 min. After 6 hours' heating at 120°C the solution was cooled to 20°C and 1.1 grams (6,0 mmol) of p-toluene sulphonic monohydrate was added. This was followed by 6 hours' heating at reflux temperature, during which 1-2 ml of a xylene/methanol mixture was removed through distillation. After cooling, the brown solution was dissolved in dichloromethane and washed with a 5% Na₂CO₃ solution in water and a 0.5M KH₂PO₄ solution in water. After drying with the aid of Na₂SO₄ and evaporation, the chemically pure product was obtained after chromatography using silica gel (eluant: toluene/ethyl acetate 2:1). 85% enantiomeric excess (determined with the aid of anisochrony in ¹H NMR, see C. Giordano et al.; J. Org. Chem. 1991, (59), 2270). The enantiomeric excess was increased to >95% through one recrystallisation from toluene. White needles. Melting point: 200-202 °C. [α]²⁰_{D} +109 (c=0.4, methanol). ¹H NMR (200 MHz, CDCl₃): 2.93 (d, 1H), 3.71 (s, 3H), 4.41 (dd, 1H), 5.02 (d, 1H), 6.74 (d, 2H), 7.03-7.21 (m, 2H), 7.29-7.44 (d+m, 3H), 7.61 (d, 1H) and 8.47 (br s, 1H). The (1S,2R)-1-(diethylamino)-2-indanol was recovered from the 0.5M KH₂PO₄ extraction solution.

### Example IX

### (2R,3S)-3-(4-Methoxyphenyl)oxirane-2-carboxylic methyl ester

A solution of 2.5 grams (6.3 mmol) of the reaction product of Example VII in 15 ml of 0.35M sodium methoxide in methanol was stirred at room temperature for 1 hour. The solution was neutralised with the aid of 40 ml of a 0.5M KH₂PO₄ solution in water and was extracted with 2*30 ml chloroform. The chloroform solution was washed with 2*30 ml of a 0.5M KH₂PO₄ solution in water. After drying (Na₂SO₄) and evaporation, 1.24 grams (95%) of (2R,3S)-3-(4-methoxyphenyl)oxirane-2-carboxylic methyl ester was obtained. Enantiomeric excess 55%. ¹H NMR (200 MHz, CDCl₃): 3.47 (d, 1H), 3.73 (s) and 3.76 (s, together 6H), 4.01 (d, 1H), 6.85 (d, 1H) and 7.18 (d, 2H). ¹³C NMR (50.31 MHz, CDCl₃) : 52.00 (q), 54.78 (q), 55.99 (d), 57.40 (d), 113.59 (d), 126.17 (s), 126.66 (d), 159.73 (s) and 168.28 (s).
After neutralisation with the aid of a 50% sodium hydroxide solution in water and extraction with the aid of chloroform, 1.30 grams (100%) of (1S,2R)-N-methyl,N-i-propyl-1-amino-2-indanol was recovered from the acid water layers.

## Claims

1. Process for preparing an optically active trans-phenylglycidic acid derivative having formula (1), 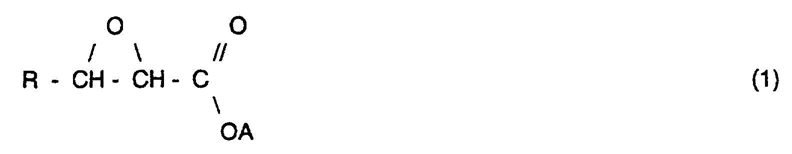 in which R represents a phenyl group, whether or not substituted, and A is derived from an optically active compound, in which an aldehyde having formula (2), 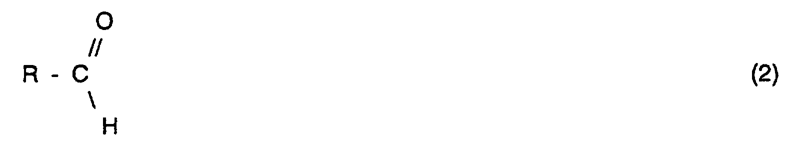 in which R is as defined above, is, in the presence of a base, brought into contact with an optically active acetyl compound having formula (3), 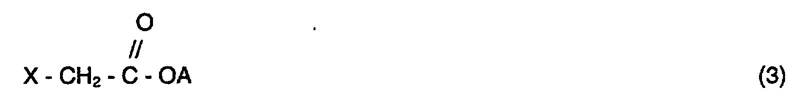 in which X represents a leaving group, characterised in that use is made of an optically active compound having formula (3), in which A is derived from an amino alcohol.

2. Process according to Claim 1, in which R represents a p-methoxyphenyl group.

3. Process according to Claim 1 or Claim 2, in which A is derived from an amino alcohol having a rigid structure.

4. Process according to Claim 3, in which A has a ring structure.

5. Process according to Claim 4, in which A is derived from an amino indanol compound having formula (4), 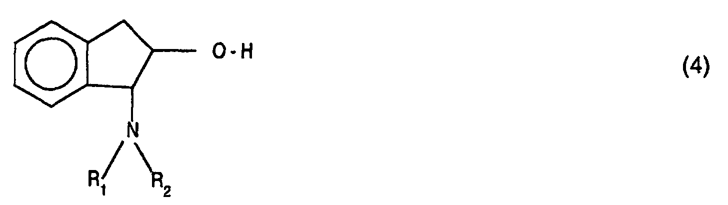 in which R₁ and R₂ represent a (hetero)alkyl, an alkenyl or (hetero)aryl group, whether or not substituted, having 1-10 C atoms, or an arylsulphonyl group, or R₁ and R₂ constitute an aromatic or aliphatic ring together with the N atoms to which they are bound.

6. Process according to Claim 5, in which R₁ and R₂ each independently of one another represent an alkyl or an alkenyl group having 1-4 C-atoms, a benzyl or a tosyl group.

7. Process according to any one of Claims 1-6, in which potassium t-butoxide is used as the base.

8. Optically active compound having formula (3), in which X represents a leaving group, A is derived from an amino alcohol having formula (4) and R₁ and R₂ represent a (hetero)alkyl, an alkenyl or (hetero)aryl group, whether or not substituted, having 1-10 C atoms, or an arylsulphonyl group or R₁ and R₂ constitute an aromatic or aliphatic ring together with the N atom to which they are bound.

9. Optically active compound according to Claim 8, in which X represents Cl or Br.

10. Optically active compound having formula (1), in which R is as defined above and A is derived from an amino alcohol having formula (4), in which R₁ and R₂ represent a (hetero)alkyl, an alkenyl or (hetero)aryl group, whether or not substituted, having 1-10 C atoms, or a arylsulphonyl group, or R₁ and R₂ constitute an aromatic or aliphatic ring together with the N atom to which they are bound.

11. Optically active compound according to any one of Claims 8-10, in which R₁ and R₂ each independently of one another represent methyl, ethyl, n-propyl, isopropyl, n-butyl, allyl, benzyl or tosyl.

12. Process for preparing a phenylglycidyl ester in which first a compound having formula (1) is prepared according to any one of claims 1-7, after which this compound is subjected to a reaction with a base and an alcohol.

13. Process according to claim 12, in which the phenylglycidyl ester is subsequently reacted with aminothiophenol.

14. Process according to any one of Claims 1-7, in which process the compound having formula (1) is subsequently subjected to a reaction with an aminothiophenol.

15. Process according to claim 13 or 14, in which subsequently the product obtained is cyclisized and optionally subsequently alkylated and/or acylated.

16. Process according to Claim 15, in which diltiazem is obtained as the benzothiazepine.

## Patentansprüche

1. Verfahren zum Herstellen eines optisch aktiven trans-Phenylepoxypropionsäurederivats der Formel (1), 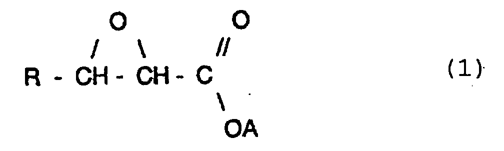 worin R eine Phenylgruppe darstellt, entweder substituiert oder nicht, und A aus einer optisch aktiven Verbindung hergeleitet wird, wobei ein Aldehyd der Formel (2), 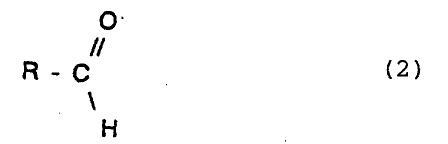 worin R wie oben definiert ist, in Anwesenheit einer Base mit einer optisch aktiven Acetylverbindung der Formel (3), 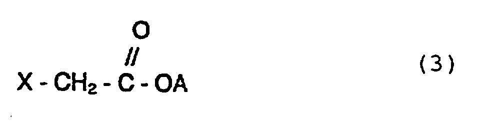 worin X eine austretende Gruppe darstellt, in Kontakt gebracht wird, dadurch gekennzeichnet, dass von einer optisch aktiven Verbindung der Formel (3) Gebrauch gemacht wird, worin A von einem Aminoalkohol hergeleitet wird.

2. Verfahren gemäss Anspruch 1, wobei R eine p-Methoxyphenylgruppe darstellt.

3. Verfahren gemäss Anspruch 1 oder Anspruch 2, wobei A von einem Aminoalkohol mit einer starren Struktur hergeleitet wird.

4. Verfahren gemäss Anspruch 3, wobei A eine Ringstruktur aufweist.

5. Verfahren gemäss Anspruch 4, wobei A von einer Aminoindanolverbindung der Formel (4) 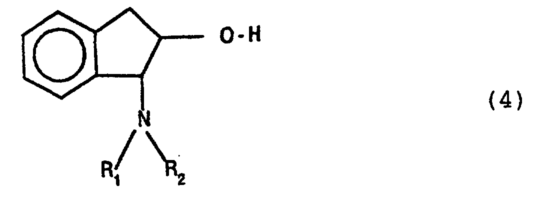 hergeleitet wird, worin R₁ und R₂ eine (Hetero)alkyl-, eine Alkenyl- oder (Hetero)arylgruppe darstellen, entweder substituiert oder nicht, mit 1-10 C-Atomen, oder eine Arylsulfonylgruppe, oder R₁ und R₂ zusammen mit den N-Atomen, an welche sie gebunden sind, einen aromatischen oder aliphatischen Ring bilden.

6. Verfahren gemäss Anspruch 5, wobei R₁ und R₂ jeweils unabhängig voneinander eine Alkyl- oder Alkenylgruppe mit 1-4 C-Atomen, eine Benzyl- oder eine Tosylgruppe darstellen.

7. Verfahren gemäss einem der Ansprüche 1-6, wobei Kalium-t-butoxid als Base verwendet wird.

8. Optisch aktive Verbindung der Formel (3), worin X eine austretende Gruppe darstellt, A aus einem Aminoalkohol der Formel (4) hergeleitet wird und R₁ und R₂ eine Hetero(alkyl)-, eine Alkenyl- oder (Hetero)arylgruppe, entweder substituiert oder nicht, mit 1-10 C-Atomen, oder eine Arylsulfonylgruppe darstellen, oder R₁ und R₂ zusammen mit den N-Atomen, an welche sie gebunden sind, einen aromatischen oder aliphatischen Ring bilden.

9. Optisch aktive Verbindung gemäss Anspruch 8, worin X für Cl oder Br steht.

10. Optisch aktive Verbindung der Formel (1), worin R wie oben definiert ist, und A aus einem Aminoalkohol der Formel (4) hergeleitet wird, worin R₁ und R₂ eine Hetero(alkyl)-, eine Alkenyl- oder (Hetero)arylgruppe, entweder substituiert oder nicht, mit 1-10 C-Atomen, oder eine Arylsulfonylgruppe darstellen, oder R₁ und R₂ zusammen mit den N-Atomen, an welche sie gebunden sind, einen aromatischen oder aliphatischen Ring bilden.

11. Optisch aktive Verbindung gemäss einem der Ansprüche 8-10, worin R₁ und R₂ jeweils unabhängig voneinander Methyl, Ethyl, n-Propyl, Isopropyl, n-Butyl, Allyl, Benzyl oder Tosyl darstellen.

12. Verfahren zum Herstellen eines Phenylglycidylesters, wobei zuerst eine Verbindung der Formel (1) gemäss einem der Ansprüche 1-7 hergestellt wird, wonach diese Verbindung einer Umsetzung mit einer Base und einem Alkohol unterworfen wird.

13. Verfahren gemäss Anspruch 12, wobei der Phenylglycidylester nachfolgend mit Aminothiophenol umgesetzt wird.

14. Verfahren gemäss einem der Ansprüche 1-7, bei welchem Verfahren die Verbindung der Formel (1) nachfolgend einer Umsetzung mit einem Aminothiophenol unterworfen wird.

15. Verfahren gemäss Anspruch 13 oder 14, bei welchem nachfolgend das erhaltene Produkt cyclisiert und gegebenenfalls nachfolgend alkyliert und/oder acyliert wird.

16. Verfahren gemäss Anspruch 15, wobei Diltiazem als das Benzothiazepin erhalten wird.

## Revendications

1. Procédé de préparation de dérivé d'acide transphénylglycidique optique-ment actif de formule (1) : 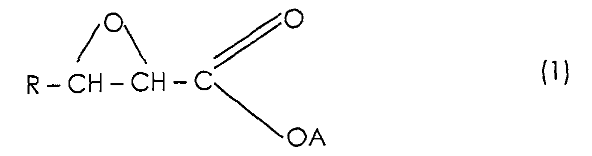 dans laquelle R représente un groupe phényle, substitué ou non et A est dérivé d'un composé optiquement actif, un aldéhyde de formule (2): 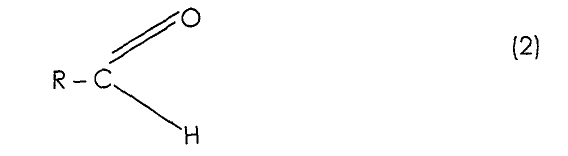 dans laquelle R est comme défini ci-dessus, étant, en présence d'une base, mis en contact avec un composé acétyle optiquement actif de formule (3) : 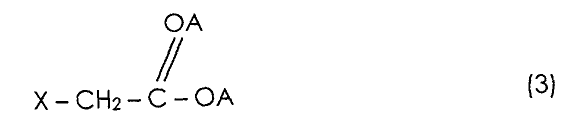 dans laquelle X représente un groupe partant, caractérisé en ce qu'on utilise un composé optiquement actif de formule (3) dans laquelle A est dérivé d'un aminoalcool.

2. Procédé selon la revendication 1, dans lequel R représente un groupe p-méthoxyphényle.

3. Procédé selon la revendication 1 ou 2, dans lequel A est dérivé d'un aminoalcool ayant une structure rigide.

4. Procédé selon la revendication 3, dans lequel A a une structure de noyau.

5. Procédé selon la revendication 4, dans lequel A est dérivé d'un composé amino-indanol de formule (4) : 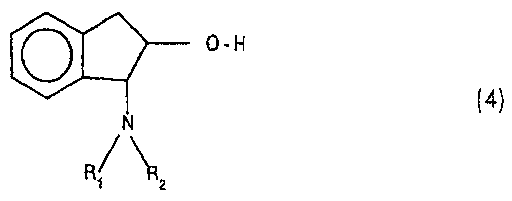 dans laquelle R₁ et R₂ représentent un (hétéro)-alkyle, un alcényle, un (hétéro)-aryle, substitué ou non, ayant 1 à 10 atomes de carbone, ou un arylsulfonyle ou R₁ et R₂ constituent un noyau aromatique ou aliphatique avec l'atome N auquel ils sont liés.

6. Procédé selon la revendication 5, dans lequel R₁ et R₂ représentent chacun indépendamment un groupe alkyle ou alcényle de 1 à 4 atomes de carbone, un groupe benzyle ou tosyle.

7. Procédé selon l'une quelconque des revendications 1 à 6, dans lequel on utilise comme base un t-butylate de potassium.

8. Composé optiquement actif de formule (3), dans lequel X représente un groupe partant, A est dérivé d'un aminoalcool de formule 4 et R₁ et R₂ représentent un groupe (hétéro)-alkyle, alcényle ou (hétéro)-aryle, substitué ou non ayant 1 à 10 atomes de carbone ou un groupe arylsulfonyle ou R₁ et R₂ constituent un noyau aromatique ou aliphatique avec l'atome N auquel ils sont liés.

9. Composé optiquement actif selon la revendication 8, dans lequel X représente Cl ou Br.

10. Composé optiquement actif de formule (1) dans laquelle R est comme défini ci-dessus et A est dérivé d'un aminoalcool de formule (4) dans laquelle R₁ et R₂ représentent un groupe (hétéro)-alkyle, alcényle ou (hétéro)-aryle, substitué ou non, ayant 1 à 10 atomes de carbone ou un groupe arylsulfonyle ou R₁ et R₂ constituent un noyau aromatique ou aliphatique avec l'atome N auquel ils sont liés.

11. Composé optiquement actif selon l'une quelconque des revendications 8 à 10, dans lequel R₁ et R₂ représentent chacun indépendamment le méthyle, l'éthyle, le n-propyle, l'isopropyle, le n-butyle, l'allyle, le benzyle ou le tosyle.

12. Procédé de préparation d'un ester phénylglycidylique dans lequel on prépare d'abord un composé de formule (1) selon l'une quelconque des revendications 1 à 7, après quoi on soumet ce composé à une réaction avec une base et un alcool.

13. Procédé selon la revendication 12, dans lequel on fait ensuite réagir l'ester phénylglycidylique avec l'aminothiophénol.

14. Procédé selon l'une quelconque des revendications 1 à 7, dans lequel le composé de formule (1) est ensuite soumis à une réaction avec un aminothiophénol.

15. Procédé selon la revendication 13 ou 14, dans lequel on cyclise ensuite le produit obtenu, puis facultativement on l'alkyle et/ou l'acyle.

16. Procédé selon la revendication 15, dans lequel on obtient un diltiazem comme benzothiazépine.
